# EUROPÄISCHE PATENTANMELDUNG

(11) **EP 4 534 010 A1**
(43) Veröffentlichungstag der Anmeldung: **09.04.2025**
(21) Anmeldenummer: 24204637.3
(22) Anmeldetag: 04.10.2024
(51) Int. Cl.: A61B 5/15, B01L 9/00

(54) **KAPILLARHALTER**

(30) Priorität: 05.10.2023 AT 508152023
(71) Anmelder: Tagtron GmbH, 2340 Mödling (AT)
(72) Erfinder: MITTERNDORFER, Fridolin, 4844 Regau (AT); SCHEFZIG, Fabian, 4844 Regau (AT); SCHODERMAYR, Christoph, 4844 Regau (AT); BÖTTCHER, Michael, 06847 Dessau-Roßlau (DE)
(74) Vertreter: SONN Patentanwälte GmbH & Co KG

(57) **Zusammenfassung**

Die Erfindung betrifft einen Kapillarhalter (1), aufweisend einen länglichen Hohlkörper (2), und einen länglichen Fixierkörper (3), der zumindest teilweise im Hohlkörper (2) angeordnet ist; wobei der Fixierkörper (3) entlang der Längsachse (4) des Hohlkörpers (2) bewegbar ist; wobei ein Mantel (6) des Hohlkörpers (2) eine erste Aussparung (9) aufweist, die eingerichtet ist, einen Abschnitt (10) einer Kapillare (11) derart aufzunehmen, dass eine Längsachse (12) der Kapillare (11) im Wesentlichen senkrecht zu einer Längsachse (4) des Hohlkörpers (2) ist; und wobei der Fixierkörper (3) eine Fixierfläche (21, 22) aufweist, die eingerichtet ist, am in der ersten Aussparung (9) aufgenommenen Abschnitt (10) der Kapillare (11) derart anzuliegen, dass die Kapillare (11) durch eine über die Fixierfläche (21, 22) auf sie wirkende Kraft an eine durch die erste Aussparung (9) im Mantel (6) des Hohlkörpers (2) gebildete Gegenfläche (19, 20) gedrückt wird, um die Kapillare (11) zwischen der Fixierfläche (21, 22) und der Gegenfläche (19, 20) zu fixieren.

## Beschreibung

Die Erfindung betrifft einen Kapillarhalter. Weiters betrifft die Erfindung eine Vorrichtung und ein Verfahren zur Abnahme einer Flüssigkeitsprobe.

Die DE 39 32 112 C2 offenbart eine Blutentnahmevorrichtung mit einer in ihrem vorderen Ende in die Vene eines Patienten einstechbaren Kanüle. An das hintere Ende der Kanüle ist ein Innenrohr angeschlossen, das sich in das Innere eines Probenröhrchens erstreckt. Zunächst wird das Innenrohr mit Blut gefüllt, und anschließend wird das Blut aus dem Innenrohr in das Probenröhrchen geleitet.

In der DE 20 2004 004 951 U1 wird eine Vorrichtung zur Entnahme von Kapillarblut offenbart, umfassend ein Blutentnahmegefäß, das an seinem vorderen Ende mit einer das Blut selbsttätig einsaugenden Kapillare ausgebildet ist. Das hintere Ende der Kapillare ist zur Ermittlung eines Probenvolumens mit einem in definierter Position eingedrückten, luftdurchlässigen, den Blutfluss automatisch stoppenden, porösen Begrenzungselement versehen.

Die DE 27 51 503 beschreibt ein Kapillargefäß zur Entnahme und Aufbewahrung von Blut. Durch die Spitze eines Einsatzes mit einem geringen Innendurchmesser wird Blut durch Kapillarwirkung eingesaugt. Das eintretende Blut kann dann durch einen Hauptteil mit einem großen Innendurchmesser in ein Aufnahmegefäß frei abfließen.

Die DE 25 58 311 A1 offenbart eine Blutsammelvorrichtung, die ein Kapillarröhrchen aufweist, das sich durch einen Deckel hindurch in ein Aufnahmegefäß erstreckt. Das Ende des Kapillarröhrchens innerhalb des Aufnahmegefäßes liegt in unmittelbarer Nähe der Innenfläche der Seitenwand des Gefäßes, sodass Blut durch das Kapillarröhrchen zur inneren Seitenwand des Aufnahmegefäßes strömen kann.

In der WO 96/14017 A1 wird ein Blutentnahmegerät offenbart, das eine Küvette, einen Kapillarhalter und eine Kapillare aufweist. Blut kann mit der Kapillare aufgenommen und anschließend in die Küvette überführt werden.

In den im Stand der Technik bekannten Vorrichtungen zur Entnahme von Blut ist die Kapillare fest mit dem Aufnahmegefäß verbunden, beispielsweise mit einem Stopfen. Vor der Blutentnahme ist folglich eine Montage der Kapillare am Aufnahmegemäß notwendig, gefolgt von einer Demontage nach der Blutentnahme. Es besteht ein Bedarf an einem Kapillarhalter, der leichter gehandhabt werden kann. Eine Aufgabe der Erfindung ist es, einen solchen Kapillarhalter zur Verfügung zu stellen.

Die Erfindung betrifft einen Kapillarhalter, aufweisend
einen länglichen Hohlkörper, und
einen länglichen Fixierkörper, der zumindest teilweise im Hohlkörper angeordnet ist,
wobei der Fixierkörper entlang der Längsachse des Hohlkörpers bewegbar ist,
wobei ein Mantel des Hohlkörpers eine erste Aussparung aufweist, die eingerichtet ist, einen Abschnitt einer Kapillare derart aufzunehmen, dass eine Längsachse der Kapillare im Wesentlichen senkrecht zu einer Längsachse des Hohlkörpers ist, und
wobei der Fixierkörper eine Fixierfläche aufweist, die eingerichtet ist, am in der ersten Aussparung aufgenommenen Abschnitt der Kapillare derart anzuliegen, dass die Kapillare durch eine über die Fixierfläche auf sie wirkende Kraft an eine durch die erste Aussparung im Mantel des Hohlkörpers gebildete Gegenfläche gedrückt wird, um die Kapillare zwischen der Fixierfläche und der Gegenfläche zu fixieren.

Die Erfindung stellt einen einfach aufgebauten und leicht herstellbaren Kapillarhalter bereit. Insbesondere braucht keine Feder vorgesehen zu werden, um den Fixierkörper entlang der Längsachse des Hohlkörpers (d.h. relativ zum Hohlkörper) zu bewegen. Dies erleichtert nachfolgend auch eine umweltfreundliche Entsorgung des Kapillarhalters, insbesondere, wenn dieser einen Karton enthält bzw. sogar aus Karton besteht. Ein Kapillarhalter enthaltend Karton ist auch deshalb vorteilhaft, weil dann ein versehentlich auf den Kapillarhalter auftreffender Flüssigkeitstropfen vom Kapillarhalter aufgesaugt (d.h. absorbiert) werden kann, sodass eine Kontamination der Umgebung oder eines Benutzers durch Hinuntertropfen des Flüssigkeitstropfens vom Kapillarhalter vermieden wird.

Der Abschnitt der Kapillare kann durch entsprechendes Bewegen des Kapillarhalters relativ zur Kapillare in der ersten Aussparung aufgenommen werden, wenn der Kapillarhalter in einem Aufnahmezustand ist. Das Bewegen kann ein drückendes und/oder ziehendes Bewegen des Fixierkörpers relativ zum Hohlkörper sein. Der in der ersten Aussparung aufgenommene Abschnitt der Kapillare ist dementsprechend innerhalb des Umfangs des Mantels des Hohlkörpers angeordnet. Unter dem Aufnahmezustand wird ein Zustand des Kapillarhalters verstanden, in welchem der Abschnitt der Kapillare in der ersten Aussparung derart aufgenommen werden kann, dass die Längsachse der Kapillare im Wesentlichen senkrecht zur Längsachse des Hohlkörpers ist. In anderen Worten ist im Aufnahmezustand der Fixierkörper relativ zum Hohlkörper derart angeordnet, dass die erste Aussparung zumindest teilweise frei bleibt, sodass ausreichend Platz zur Aufnahme des Abschnitts der Kapillare zur Verfügung steht.

Nach der Aufnahme des Abschnitts der Kapillare im Kapillarhalter kann der Kapillarhalter in einen Fixierzustand gebracht werden. Unter dem Fixierzustand des Kapillarhalters wird ein Zustand verstanden, in welchem die Fixierfläche derart am in der ersten Aussparung aufgenommenen Abschnitt der Kapillare anliegt, dass die Kapillare durch eine über die Fixierfläche auf sie wirkende Kraft, insbesondere eine Normalkraft, an eine durch die erste Aussparung im Mantel des Hohlkörpers gebildete Gegenfläche gedrückt wird. Dementsprechend wird die Kapillare im Fixierzustand des Kapillarhalters kraftschlüssig gehalten, indem sie zwischen der Fixierfläche und der Gegenfläche eingeklemmt und dadurch in einer bestimmten Position relativ zum Kapillarhalter fixiert wird.

Mit dem Kapillarhalter gemäß der Erfindung kann eine Flüssigkeitsprobe, insbesondere eine Blutprobe, abgenommen werden, ohne dass die Kapillare mit der Hand eines Benutzers direkt berührt werden muss. Die Kapillare kann z.B. in einem Kapillarhalter bereitgestellt werden. Durch entsprechendes Bewegen des Kapillarhalters relativ zur Kapillare kann der Abschnitt der Kapillare in der ersten Aussparung aufgenommen werden, und kann die Kapillare anschließend fixiert werden, indem der Kapillarhalter in den Fixierzustand gebracht wird. Auch kann der Kapillarhalter mit nur einer Hand bedient werden, insbesondere kann er einhändig vom Aufnahmezustand in den Fixierzustand gebracht werden und umgekehrt. Der Kapillarhalter gemäß der Erfindung ist daher leicht handhabbar. Die durch den Kapillarhalter gehaltene Kapillare kann so eine Flüssigkeitsprobe, z.B. Blut eines Patienten, aufnehmen. Kontaminationen durch die Flüssigkeitsprobe am Benutzer, der den Kapillarhalter hält, werden so vermieden.

Die Aufnahme des Abschnitts der Kapillare in der ersten Aussparung derart, dass die Längsachse der Kapillare im Wesentlichen senkrecht zur Längsachse des Hohlkörpers ist, hat den Vorteil, dass ein Ende der Kapillare den größtmöglichen Normalabstand zum Kapillarhalter aufweisen kann, insbesondere das Ende, das mit der Flüssigkeitsprobe in Kontakt kommt. Dadurch kann die Gefahr einer Kontamination des Kapillarhalters verringert werden. Auch wird dadurch die Handhabung erleichtert, weil der Kapillarhalter im Fixierzustand von einem Benutzer dann mit vergleichsweise geringem Abstand zum Abschnitt der Kapillare, die in der ersten Aussparung aufgenommen ist, außen am Hohlkörper gehalten werden kann, ohne dass eine unabsichtliche Berührung mit der Kapillare erfolgt.

Die Handhabung des Kapillarhalter kann weiters durch eine Haftoberfläche an der Außenseite des Hohlkörpers verbessert werden. Der Hohlkörper - und damit der gesamte Kapillarhalter - werden von einem Benutzer an der Außenseite des Hohlkörpers in die Hand genommen und mit Fingern bedient. Bei der oben beschriebenen Benutzung mit einer Hand halten wenige Finger den Hohlkörper und andere Finger verschieben den Fixierkörper. Es kann eine Haftoberfläche an der Außenseite des Hohlkörpers vorgesehen werden. Diese Haftoberfläche verringert oder verhindert ein Verrutschen der Finger des Benutzers an der Oberfläche des Hohlkörpers, insbesondere bei einhändiger Benutzung. Die Haftoberfläche kann eine raue Oberfläche, eine der mehrere Rillen, eine oder mehrere Kanten oder beliebige Vertiefungen und/oder Erhöhungen sein. Dies erhöht die Grifffestigkeit - auch mit nur wenigen, z.B. 2, Fingern des Benutzers. Ein spezielles Beispiel einer Haftoberfläche ist eine äußere Ummantelung, z.B. die mit dem Hohlkörper fest verbunden ist. Die äußere Ummantelung kann an ihrem Ende eine Kante haben, an der die Finger des Benutzers Halt finden. Eine äußere Ummantelung ist vorzugsweise eine Karton- oder Papierschicht. Vorzugsweise ist die Haftoberfläche in Längsachse des Hohlkörpers in der von der Aussparung des Hohlkörpers entfernten Hälfte des Hohlkörpers positioniert.

Der Fixierkörper ist relativ zum Hohlkörper vorzugsweise derart angeordnet, dass eine Längsachse des Fixierkörpers mit der Längsachse des Hohlkörpers zusammenfällt. Dies vereinfacht das Bewegen des Fixierkörpers relativ zum Hohlkörper.

Der Fixierkörper ist vorzugsweise länger als der Hohlkörper. Dies vereinfacht ein Bewegen des Fixierkörpers relativ zum Hohlkörper. Insbesondere kann der Fixierkörper dann an einem aus dem Hohlkörper herausragenden Ende gehalten werden, um ihn relativ zum Hohlkörper zu bewegen, insbesondere, um den Kapillarhalter in den Aufnahmezustand oder den Fixierzustand zu bringen.

Der Fixierkörper, insbesondere eine Außenseite des Mantels des Fixierkörpers, ist vorzugsweise eingerichtet, zumindest teilweise an einer Innenseite des Mantels des Hohlkörpers anzuliegen. Der Fixierkörper kann dann einerseits entlang der Längsachse des Hohlkörpers bewegbar sein, andererseits können der Hohlkörper und der Fixierkörper relativ zueinander reibschlüssig in einer bestimmten Position gehalten werden, insbesondere im Aufnahmezustand oder im Fixierzustand des Kapillarhalters. Dies kann die Handhabung des Kapillarhalters vereinfachen, insbesondere braucht im Fixierzustand keine Kraft von außen (durch einen Benutzer) auf den Fixierkörper aufgebracht zu werden, um die zum Fixieren der Kapillare notwendige Kraft zu erzeugen. Vielmehr kann diese Kraft bereits durch die Reibungskraft, die zwischen dem Fixierkörper und dem Hohlkörper wirkt, erzeugt werden. Um den Fixierkörper relativ zum Hohlkörper zu bewegen (durch Schieben, Ziehen) und ihn vom Aufnahmezustand in den Fixierzustand zu bringen (oder umgekehrt), muss diese Reibungskraft dann überwunden werden. Dies kann durch Aufbringen einer Kraft von außen (durch den Benutzer) auf den Fixierkörper erfolgen.

Vorzugsweise weist eine Außenseite des Mantels des Fixierkörpers und/oder eine Innenseite des Mantels des Hohlkörpers ein Material mit einem ausreichend hohen Haftreibungskoeffizienten auf, um aufgrund der dadurch erzeugten Reibungskraft zwischen dem Fixierkörper und dem Hohlkörper eine bestimmte Position des Fixierkörpers relativ zum Hohlkörper beizubehalten, sofern keine Kraft von außen auf den Kapillarhalter wirkt. Ein derartiges Material kann Karton enthalten.

Der Hohlkörper und/oder der Fixierkörper enthalten vorzugsweise einen Karton. Dadurch kann der Kapillarhalter nachhaltig produziert und nach seiner Verwendung recycelt werden. Insbesondere können der Hohlkörper und/oder der Fixierkörper Zellstoff und/oder Zellulose enthalten. Vorzugsweise bestehen der Hohlkörper und/oder der Fixierkörper aus einem Karton. Dementsprechend sind der Hohlkörper und/oder der Fixierkörper vorzugsweise unbeschichtet. Wenn der Hohlkörper aus Karton besteht, kann ein Flüssigkeitstropfen, der versehentlich auf den Kapillarhalter gelangt, durch den Hohlkörper aufgesaugt werden. Dementsprechend kann er nicht am Kapillarhalter entlanglaufen bzw. hinuntertropfen, sodass eine Kontamination eines Benutzers oder der Umgebung vermieden werden kann. Der kontaminierte Kapillarhalter kann nach seiner Verwendung fachgerecht entsorgt werden. Vorzugsweise ist der Kapillarhalter nahe der ersten Aussparung unbeschichtet.

Der Hohlkörper weist vorzugsweise eine Wanddicke im Bereich von 300 bis 3000 µm auf, bevorzugter von 500 bis 2000 pm, noch bevorzugter von 600 bis 900 pm, noch bevorzugter von 700 bis 800 um. Dadurch kann der Hohlzylinder eine gute Formstabilität aufweisen. Auch kann dann eine ausreichend große Gegenfläche gebildet werden, um die Kapillare im Fixierzustand des Kapillarhalters gut zu fixieren.

Vorzugsweise ist ein Außendurchmesser des Hohlkörpers mindestens das 1-fache des Außendurchmessers der Kapillare, bevorzugter mindestens das 1,5-fache, noch bevorzugter mindestens das 2-fache (wenn der Hohlkörper und die Kapillare eine runde oder ovale Querschnittsfläche haben; bei einer ovalen Querschnittsfläche wird unter dem Außendurchmesser der minimale Außendurchmesser verstanden); bzw. ist vorzugsweise eine Seite der Querschnittsfläche des Hohlkörpers mindestens das 1-fache einer Seite der Querschnittsfläche der Kapillare, bevorzugter mindestens das 1,5-fache, noch bevorzugter mindestens das 2-fache (wenn der Hohlkörper und die Kapillare quaderförmig sind). Dann kann eine ausreichend große erste Aussparung im Hohlkörper vorgesehen werden, um den Abschnitt der Kapillare aufnehmen zu können, insbesondere derart, dass dieser Abschnitt der Kapillare ihren gesamten Umfang beinhaltet; dann kann im Fixierzustand eine Fixierung der Kapillare entlang ihres gesamten Umfangs erfolgen. Vorzugsweise weist der Hohlkörper einen Außendurchmesser (bzw. eine Seite der Querschnittsfläche) im Bereich von 2 bis 10 mm auf, bevorzugter von 3 bis 7 mm.

Vorzugsweise weisen der Hohlkörper und/oder der Fixierkörper eine konstante Querschnittsfläche über die Länge auf. Dann kann der Fixierkörper entlang der Länge des Hohlkörpers in jeder beliebigen Position angeordnet werden, und weisen der Fixierkörper und der Hohlkörper entlang der Länge des Hohlkörpers, entlang derer der Fixierkörper im Hohlkörper angeordnet ist, stets denselben Normalabstand zueinander auf.

Der Hohlkörper und/oder der Fixierkörper weisen vorzugsweise eine ovale oder runde Querschnittsfläche auf. Besonders bevorzugt ist es, wenn der Hohlkörper eine ovale und der Fixierkörper eine runde Querschnittsfläche aufweist; oder wenn der Hohlkörper eine runde und der Fixierkörper eine ovale Querschnittsfläche aufweist; oder wenn sowohl der Hohlkörper als auch der Fixierkörper eine ovale Querschnittsfläche aufweisen. Dann kann eine Verdrehung des Fixierkörpers relativ zum Hohlkörper vermieden werden. Somit kann sichergestellt werden, dass die Fixierfläche relativ zur Gegenfläche derart angeordnet ist, dass die Kapillare im Fixierzustand des Kapillarhalters stabil fixiert werden kann.

Der Fixierkörper kann ein Vollkörper sein, oder er kann hohl sein. Wenn der Fixierkörper hohl ist, weist er vorzugsweise eine Wanddicke im Bereich von 300 bis 3000 µm auf, bevorzugter von 500 bis 2000 pm, noch bevorzugter von 600 bis 900 pm, noch bevorzugter von 700 bis 800 µm. Dann kann der Fixierkörper eine gute Formstabilität aufweisen. Auch kann dann eine ausreichend große Fixierfläche gebildet werden, um die Kapillare im Fixierzustand des Kapillarhalters gut zu fixieren.

Der Fixierkörper weist vorzugsweise eine geschlossene Endfläche auf, bevorzugter zwei geschlossene Endflächen. Das bedeutet, dass die Enden des Fixierkörpers keine Öffnungen aufweisen, was die Handhabung des Kapillarhalters erleichtern kann. Insbesondere kann die Kapillare dann nicht versehentlich durch eines der beiden Enden des Fixierkörpers in das Innere des Fixierkörpers eingefädelt werden, denn in dieser nicht bestimmungsgemäßen Position relativ zum Kapillarhalter könnte sie nicht fixiert werden.

Die Gegenfläche wird durch die erste Aussparung im Mantel des Hohlkörpers gebildet. Sie weist vorzugsweise zwei Teilbereiche auf, die an einander gegenüberliegenden Seiten an einer durch die erste Aussparung im Mantel gebildeten Fläche angeordnet sind, wobei die Teilbereiche der Gegenfläche zumindest teilweise in einer gemeinsamen Ebene liegen, die senkrecht zur Längsachse des Hohlkörpers ist. Jeder Teilbereich der Gegenfläche kann dann zumindest teilweise am in der ersten Aussparung aufgenommenen Abschnitt der Kapillare anliegen, wenn dieser Abschnitt in der ersten Aussparung derart aufgenommen ist, dass eine Längsachse der Kapillare im Wesentlichen senkrecht zur Längsachse des Hohlkörpers ist. Dadurch kann der Abschnitt der Kapillare gleichmäßiger und stabiler fixiert werden. Wenn die Teilbereiche gekrümmt sind, kann auch nur eine Linie jedes Teilbereichs in der gemeinsamen Ebene liegen, die senkrecht zur Längsachse des Hohlkörpers ist. Mit der Formulierung, dass die Teilbereiche an einander gegenüberliegenden Seiten an einer durch die erste Aussparung im Mantel gebildeten Fläche angeordnet sind, ist gemeint, dass die Teilbereiche derart angeordnet sind, dass eine Kapillare, die vom Kapillarhalter bestimmungsgemäß gehalten wird, zugleich an beiden dieser Teilbereiche anliegen kann.

Bevorzugt weist der Kapillarhalter zwei Gegenflächen auf, die an einander gegenüberliegenden Seiten der durch die erste Aussparung im Mantel des Hohlkörpers gebildeten Fläche angeordnet sind, wobei die erste Gegenfläche zumindest teilweise in einer ersten Ebene liegt, wobei die zweite Gegenfläche zumindest teilweise in einer zweiten Ebene liegt, und wobei die erste Ebene und die zweite Ebene senkrecht zur Längsachse des Hohlkörpers und parallel zueinander sind. Die Kapillare kann im Fixierzustand dann entweder an die erste Gegenfläche oder an die zweite Gegenfläche gedrückt werden, unter anderem je nachdem, wie der Fixierkörper im Aufnahmezustand relativ zum Hohlkörper positioniert ist, und entsprechend geschoben oder gezogen werden muss, um den Kapillarhalter in den Fixierzustand zu bringen. Die zwei Gegenflächen können eine voneinander verschiedene Geometrie aufweisen. Beispielsweise kann die erste Gegenfläche gekrümmt sein und die zweite Gegenfläche kann gerade sein. Dementsprechend kann der Kapillarhalter eingerichtet sein, Kapillaren verschiedener Geometrie zu halten, etwa quaderförmige und zylinderförmige Kapillaren.

Die Fixierfläche kann an einer Endfläche des Fixierkörpers angeordnet sein. Die Fixierfläche kann an einer beliebigen Stelle an der Endfläche angeordnet sein, solange sie im Fixierzustand am in der ersten Aussparung aufgenommenen Abschnitt der Kapillare anliegen und die Kapillare an die Gegenfläche drücken kann, um sie zu fixieren. Die Fixierfläche kann an einer Innenseite oder an einer Außenseite der Endfläche angeordnet sein. Auch kann sowohl die Innenseite als auch die Außenseite der Endfläche je eine Fixierfläche aufweisen, und/oder können beide Endflächen je eine und/oder je zwei Fixierfläche(n) aufweisen.

Der Fixierkörper kann eine zweite Aussparung aufweisen, die eingerichtet ist, einen Teil des in der ersten Aussparung aufgenommenen Abschnitts der Kapillare aufzunehmen. Dabei kann eine durch die zweite Aussparung gebildete Fläche des Fixierkörpers die Fixierfläche aufweisen. Diese Fixierfläche kann zusätzlich oder alternativ zu einer an einer Endfläche des Fixierkörpers angeordneten Fixierfläche vorgesehen sein.

Wenn die durch die zweite Aussparung im Fixierkörper gebildete Fläche die Fixierfläche aufweist, weist die Fixierfläche vorzugsweise zwei Teilbereiche auf, die an einander gegenüberliegenden Seiten dieser Fläche angeordnet sind, wobei die Teilbereiche der Fixierfläche zumindest teilweise in einer gemeinsamen Ebene liegen, die senkrecht zur Längsachse des Hohlkörpers ist. Jeder Teilbereich der Fixierfläche kann dann zumindest teilweise am in der ersten Aussparung aufgenommenen Abschnitt der Kapillare anliegen, wenn die Kapillare in der ersten Aussparung derart aufgenommen ist, dass eine Längsachse der Kapillare im Wesentlichen senkrecht zur Längsachse des Hohlkörpers ist. Dadurch kann der Abschnitt der Kapillare gleichmäßiger und stabiler fixiert werden. Wenn die Teilbereiche gekrümmt sind, kann auch nur eine Linie jedes Teilbereichs in der gemeinsamen Ebene liegen, die senkrecht zur Längsachse des Hohlkörpers ist. Mit der Formulierung, dass die Teilbereiche an einander gegenüberliegenden Seiten der Fixierfläche angeordnet sind, ist gemeint, dass die Teilbereiche derart angeordnet sind, dass eine Kapillare, die vom Kapillarhalter bestimmungsgemäß gehalten wird, zugleich an beiden dieser Teilbereiche anliegen kann.

Bevorzugt weist der Kapillarhalter zwei Fixierflächen auf, die an einander gegenüberliegenden Seiten der durch die zweite Aussparung im Fixierkörper gebildeten Fläche angeordnet sind, wobei die erste Fixierfläche zumindest teilweise in einer ersten Ebene liegt, wobei die zweite Fixierfläche zumindest teilweise in einer zweiten Ebene liegt, und wobei die erste Ebene und die zweite Ebene senkrecht zur Längsachse des Hohlkörpers und parallel zueinander sind. Die Kapillare kann im Fixierzustand dann entweder an die erste Fixierfläche oder an die zweite Fixierfläche gedrückt werden, unter anderem je nachdem, wie der Fixierkörper im Aufnahmezustand relativ zum Hohlkörper positioniert ist. Die zwei Fixierflächen können eine voneinander verschiedene Geometrie aufweisen. Beispielsweise kann die erste Fixierfläche gekrümmt sein und die zweite Fixierfläche kann gerade sein. Dementsprechend kann der Kapillarhalter eingerichtet sein, Kapillaren verschiedener Geometrie zu halten, etwa quaderförmige und zylinderförmige Kapillaren.

Wenn der Fixierkörper eine zweite Aussparung aufweist und die Fixierfläche an einer durch die zweite Aussparung gebildeten Fläche angeordnet ist, ist es bevorzugt, wenn eine Endfläche des Fixierkörpers und eine Endfläche des Hohlkörpers im Aufnahmezustand des Kapillarhalters in derselben Ebene liegen. Dadurch lässt sich der Kapillarhalter durch Bewegen des Fixierkörpers relativ zum Hohlkörper rasch und einfach in den Aufnahmezustand bringen. Insbesondere, wenn der Hohlkörper und der Fixierkörper relativ zueinander reibschlüssig in einer bestimmten Position gehalten werden, könnte es ansonsten schwieriger sein, den Fixierkörper relativ zum Hohlkörper exakt derart zu positionieren, dass der Kapillarhalter im Aufnahmezustand ist.

Vorzugsweise ist die Fixierfläche und/oder die Gegenfläche zumindest teilweise normal zur Längsachse des Hohlkörpers. Dann kann die Kraft für die Fixierung der Kapillare im Fixierzustand weiter erhöht werden.

Vorzugsweise erstrecken sich die erste Aussparung und die zweite Aussparung in radialer Richtung über zumindest die Hälfte der Querschnittsfläche des Fixierkörpers. Dann kann der in der ersten Aussparung aufgenommene Abschnitt der Kapillare ihren gesamten Umfang beinhalten; d.h. die Kapillare kann dann entlang ihres gesamten Umfangs fixiert werden.

Die erste Aussparung ist vorzugsweise von Enden des Hohlkörpers beabstandet. Dies kann die Fixierung der Kapillare im Fixierzustand weiter verbessern.

Vorzugsweise korrespondiert die erste Aussparung mit der zweiten Aussparung. Bei entsprechender Anordnung des Fixierkörpers relativ zum Hohlkörper können die zwei Aussparungen im Aufnahmezustand des Kapillarhalters dann derart angeordnet werden, dass sie bündig übereinanderliegen. Dadurch können die gegebenen Abmessungen der Aussparungen optimal ausgenutzt werden, um eine einfache Aufnahme des Abschnitts der Kapillare zu gewährleisten.

Vorzugsweise weist die erste Aussparung und/oder die zweite Aussparung die Form eines Halbzylinders, eines Zylinders, eines Halbovalzylinders, eines Ovalzylinders, eines Quaders, eines Würfels oder eine Kombination davon auf. Die Geometrie der Aussparungen, insbesondere die Geometrie der Fixierfläche und der Gegenfläche, kann an die Geometrie der verwendeten Kapillare angepasst werden, sodass die Kapillare gut im Kapillarhalter fixiert werden kann. Beispielsweise können die Fixierfläche und die Gegenfläche jeweils an einer Seite eines durch die beiden Aussparungen jeweils gebildeten Quaders oder Würfels vorgesehen sein, wenn die Kapillare quaderförmig ist, wobei diese Seiten des Quaders oder Würfels vorzugsweise normal zur Längsachse des Hohlkörpers sind. Wird hingegen eine zylinderförmige Kapillare verwendet, können die Fixierfläche und die Gegenfläche jeweils an einem gekrümmten Abschnitt eines durch die beiden Aussparungen jeweils gebildeten Halbzylinders, eines Zylinders, eines Halbovalzylinders oder eines Ovalzylinders vorgesehen sein.

Der Begriff "Ovalzylinder" schließt Ausführungsformen ein, die zwei Halbzylinder derselben Größe aufweisen, zwischen denen ein Quader eingefügt ist, wobei die Länge der Seitenflächen des Quaders, die an der geraden (d.h. nicht gekrümmten) Seitenfläche des jeweiligen Halbzylinders anliegen, dem Durchmesser der Halbzylinder entspricht. Die gekrümmten Seitenflächen des Halbzylinders sind dementsprechend umfangsseitig (d.h. außenliegend) angeordnet.

Weist die erste Aussparung und/oder die zweite Aussparung die Form eines Halbovalzylinders und/oder Ovalzylinders auf, ist ein gerader Abschnitt des Umfangs des Halbovalzylinders und/oder des Ovalzylinders vorzugsweise parallel zur Längsachse des Hohlkörpers. Dann kann die durch die erste Aussparung im Mantel des Hohlkörpers gebildete Gegenfläche an einem ersten gekrümmten Abschnitt des Umfangs des durch die erste Aussparung gebildeten Halbovalzylinders oder Ovalzylinders angeordnet sein; und/oder die Fixierfläche kann an einem zweiten gekrümmten Abschnitt des Umfangs des durch die zweite Aussparung gebildeten Halbovalzylinders oder Ovalzylinders angeordnet sein. Vorzugsweise ist ein Krümmungsradius des ersten und/oder zweiten gekrümmten Abschnitts zumindest gleich groß wie der Radius der Kapillare (bei Verwendung einer zylinderförmigen Kapillare). Hierdurch ist eine gute Fixierung der Kapillare möglich.

Vorzugsweise ist die erste Aussparung und/oder die zweite Aussparung derart ausgebildet, dass der Mantel des Hohlkörpers und/oder der Mantel des Fixierkörpers einen Vorsprung aufweist. Dementsprechend weist die erste Aussparung und/oder die zweite Aussparung in mantelnahen Bereichen eine geringere Größe auf als in achsnahen Bereichen. Durch den Vorsprung kann die Kapillare im Fixierzustand zusätzlich fixiert werden, da sie besser daran gehindert werden kann, aus dem Kapillarhalter herauszufallen oder herausgedrückt zu werden.

Die Erfindung betrifft weiters eine Vorrichtung zur Abnahme einer Flüssigkeitsprobe, insbesondere einer Blutprobe, aufweisend den Kapillarhalter gemäß der Erfindung und eine Kapillare. Die Kapillare ist im Fixierzustand des Kapillarhalters derart in der ersten Aussparung aufgenommen, dass eine Längsachse der Kapillare im Wesentlichen senkrecht zu einer Längsachse des Hohlkörpers ist. Weiters liegt im Fixierzustand die Fixierfläche am in der ersten Aussparung aufgenommenen Abschnitt der Kapillare derart an, dass die Kapillare durch eine über die Fixierfläche auf sie wirkende Kraft, insbesondere Normalkraft, an die durch die erste Aussparung im Mantel des Hohlkörpers gebildete Gegenfläche gedrückt wird.

Die Erfindung betrifft auch ein Verfahren zur Fixierung einer Kapillare mit dem Kapillarhalter gemäß der Erfindung, umfassend die Schritte
(a) Bereitstellen des Kapillarhalters im Aufnahmezustand,
(b) Aufnehmen des Abschnitts der Kapillare in der ersten Aussparung, und
(c) Bewegen des Fixierkörpers relativ zum Hohlkörper, um den Kapillarhalter in einen Fixierzustand zu bringen.

Die Erfindung betrifft auch ein Verfahren zum Abnehmen einer Flüssigkeitsprobe, insbesondere Blut, umfassend die vorhergehend genannten Schritte (a) bis (c), sowie Schritt (d): Abnehmen der Flüssigkeitsprobe mit der Kapillare. Die Flüssigkeitsprobe, insbesondere Blut, kann durch Punktion bereitgestellt werden, etwa durch Punktion einer Fingerkuppe.

Das Verfahren zum Abnehmen der Flüssigkeitsprobe umfasst vorzugsweise weiters Schritt (e) : Einfüllen der abgenommenen Flüssigkeitsprobe in ein Aufnahmegefäß. Dies kann berührungslos erfolgen, d.h. ohne eine Berührung des Aufnahmegefäßes durch die Kapillare. Nach Schritt (e) kann die mit der Flüssigkeitsprobe, kontaminierte Kapillare entsorgt werden.

Der Kapillarhalter kann zwei Mal oder öfter zur Abnahme einer Flüssigkeitsprobe desselben Patienten verwendet werden, insbesondere bis zu vier Mal. Nach seiner bestimmungsgemäßen Verwendung kann der Kapillarhalter entsorgt werden. Insbesondere, wenn der Kapillarhalter aus Karton besteht, gestaltet sich die Entsorgung als nachhaltig, da nur eine Abfallsorte anfällt.

Der Fixierkörper und/oder der Hohlkörper können unter Verwendung von Stanzen, Hochziehen, Tiefziehen, Drucken, Schneiden, Prägen, Lasern, Rillen oder einer Kombination davon hergestellt werden. Der Fixierkörper und/oder der Hohlkörper können anschließend derart gefaltet und/oder zusammengerollt werden, dass der Fixierkörper zumindest teilweise im Hohlkörper angeordnet werden kann.

Für die Zwecke dieser Offenbarung beziehen sich Angaben wie "Hinuntertropfen", etc. auf den bestimmungsgemäßen Betriebszustand des Kapillarhalters, d.h. auf einen Zustand, in welchem der Kapillarhalter derart ausgerichtet ist, dass eine Öffnung einer von ihm gehaltenen Kapillare in einer im Wesentlichen horizontalen Ebene liegt.

Die Erfindung betrifft insbesondere die folgenden Ausführungsformen:
1. Kapillarhalter, aufweisend
   einen länglichen Hohlkörper, und
   einen länglichen Fixierkörper, der zumindest teilweise im Hohlkörper angeordnet ist,
   wobei der Fixierkörper entlang der Längsachse des Hohlkörpers bewegbar ist,
   wobei ein Mantel des Hohlkörpers eine erste Aussparung aufweist, die eingerichtet ist, einen Abschnitt einer Kapillare derart aufzunehmen, dass eine Längsachse der Kapillare im Wesentlichen senkrecht zu einer Längsachse des Hohlkörpers ist, und
   wobei der Fixierkörper eine Fixierfläche aufweist, die eingerichtet ist, am in der ersten Aussparung aufgenommenen Abschnitt der Kapillare derart anzuliegen, dass die Kapillare durch eine über die Fixierfläche auf sie wirkende Kraft an eine durch die erste Aussparung im Mantel des Hohlkörpers gebildete Gegenfläche gedrückt wird, um die Kapillare zwischen der Fixierfläche und der Gegenfläche zu fixieren.
2. Kapillarhalter nach Ausführungsform 1, wobei der Fixierkörper relativ zum Hohlkörper derart angeordnet ist, dass eine Längsachse des Fixierkörpers mit der Längsachse des Hohlkörpers zusammenfällt.
3. Kapillarhalter nach Ausführungsform 1 oder 2, wobei der Fixierkörper länger ist als der Hohlkörper.
4. Kapillarhalter nach einer der Ausführungsformen 1 bis 3, wobei der Fixierkörper, insbesondere eine Außenseite eines Mantels des Fixierkörpers, eingerichtet ist, zumindest teilweise an einer Innenseite des Mantels des Hohlkörpers anzuliegen.
5. Kapillarhalter nach einer der Ausführungsformen 1 bis 4, wobei der Hohlkörper und/oder der Fixierkörper einen Karton enthalten, vorzugsweise wobei der Hohlkörper und/oder der Fixierkörper aus einem Karton bestehen.
6. Kapillarhalter nach einer der Ausführungsformen 1 bis 5, wobei der Hohlkörper eine Wanddicke im Bereich von 300 bis 3000 µm aufweist, vorzugweise von 500 bis 2000 µm, bevorzugter von 600 bis 900 pm, noch bevorzugter von 700 bis 800 µm.
7. Kapillarhalter nach einer der Ausführungsformen 1 bis 6, wobei ein Außendurchmesser des Hohlkörpers mindestens das 1-fache des Außendurchmessers der Kapillare ist, bevorzugter mindestens das 1,5-fache, noch bevorzugter mindestens das 2-fache.
8. Kapillarhalter nach einer der Ausführungsformen 1 bis 7, wobei der Hohlkörper einen Außendurchmesser im Bereich von 2 bis 10 mm aufweist, bevorzugter von 3 bis 7 mm.
9. Kapillarhalter nach einer der Ausführungsformen 1 bis 8, wobei der Hohlkörper und/oder der Fixierkörper eine konstante Querschnittsfläche über ihre Länge aufweisen.
10. Kapillarhalter nach einer der Ausführungsformen 1 bis 9, wobei der Hohlkörper und/oder der Fixierkörper eine ovale oder runde Querschnittsfläche aufweisen.
11. Kapillarhalter nach einer der Ausführungsformen 1 bis 10, wobei der Fixierkörper hohl ist, vorzugsweise wobei der Fixierkörper eine Wanddicke im Bereich von 300 bis 3000 µm aufweist, bevorzugter von 500 bis 2000 pm, noch bevorzugter von 600 bis 900 pm, noch bevorzugter von 700 bis 800 µm.
12. Kapillarhalter nach einer der Ausführungsformen 1 bis 11, wobei der Fixierkörper eine geschlossene Endfläche aufweist, bevorzugter zwei geschlossene Endflächen.
13. Kapillarhalter nach einer der Ausführungsformen 1 bis 12, wobei die Gegenfläche zwei Teilbereiche aufweist, die an einander gegenüberliegenden Seiten an einer durch die erste Aussparung im Mantel gebildeten Fläche angeordnet sind, wobei die Teilbereiche der Gegenfläche zumindest teilweise in einer gemeinsamen Ebene liegen, die senkrecht zur Längsachse des Hohlkörpers ist.
14. Kapillarhalter nach einer der Ausführungsformen 1 bis 13, aufweisend zwei Gegenflächen, die an einander gegenüberliegenden Seiten der durch die erste Aussparung im Mantel des Hohlkörpers gebildeten Fläche angeordnet sind, wobei die erste Gegenfläche zumindest teilweise in einer ersten Ebene liegt, wobei die zweite Gegenfläche zumindest teilweise in einer zweiten Ebene liegt, und wobei die erste Ebene und die zweite Ebene senkrecht zur Längsachse des Hohlkörpers und parallel zueinander sind.
15. Kapillarhalter nach Ausführungsform 14, wobei die zwei Gegenflächen eine voneinander verschiedene Geometrie aufweisen, vorzugsweise wobei die erste Gegenfläche gekrümmt ist und die zweite Gegenfläche gerade ist.
16. Kapillarhalter nach einer der Ausführungsformen 1 bis 15, wobei die Fixierfläche an einer Endfläche des Fixierkörpers angeordnet ist, insbesondere an einer Außenseite und/oder an einer Innenseite der Endfläche.
17. Kapillarhalter nach einer der Ausführungsformen 1 bis 16, wobei der Fixierkörper eine zweite Aussparung aufweist, die eingerichtet ist, einen Teil des in der ersten Aussparung aufgenommenen Abschnitts der Kapillare aufzunehmen.
18. Kapillarhalter nach Ausführungsform 17, wobei eine durch die zweite Aussparung gebildete Fläche des Fixierkörpers die Fixierfläche aufweist.
19. Kapillarhalter nach Ausführungsform 17 oder 18, wobei die Fixierfläche zwei Teilbereiche aufweist, die an einander gegenüberliegenden Seiten der durch die zweite Aussparung im Fixierkörper gebildeten Fläche angeordnet sind, wobei die Teilbereiche der Fixierfläche zumindest teilweise in einer gemeinsamen Ebene liegen, die senkrecht zur Längsachse des Hohlkörpers ist.
20. Kapillarhalter nach einer der Ausführungsformen 17 bis 19, aufweisend zwei Fixierflächen, die an einander gegenüberliegenden Seiten der durch die zweite Aussparung im Fixierkörper gebildeten Fläche angeordnet sind, wobei die erste Fixierfläche zumindest teilweise in einer ersten Ebene liegt, wobei die zweite Fixierfläche zumindest teilweise in einer zweiten Ebene liegt, und wobei die erste Ebene und die zweite Ebene senkrecht zur Längsachse des Hohlkörpers und parallel zueinander sind.
21. Kapillarhalter nach einer der Ausführungsformen 1 bis 20, wobei eine Endfläche des Fixierkörpers und eine Endfläche des Hohlkörpers im Aufnahmezustand des Kapillarhalters in derselben Ebene liegen.
22. Kapillarhalter nach einer der Ausführungsformen 1 bis 21, wobei die Fixierfläche und/oder die Gegenfläche zumindest teilweise normal zur Längsachse des Hohlkörpers sind.
23. Kapillarhalter nach einer der Ausführungsformen 17 bis 22, wobei sich die erste Aussparung und/oder die zweite Aussparung in radialer Richtung über zumindest die Hälfte der Querschnittsfläche des Fixierkörpers erstrecken.
24. Kapillarhalter nach einer der Ausführungsformen 1 bis 23, wobei die erste Aussparung von Enden des Hohlkörpers beabstandet ist.
25. Kapillarhalter nach einer der Ausführungsformen 17 bis 24, wobei die erste Aussparung mit der zweiten Aussparung korrespondiert.
26. Kapillarhalter nach einer der Ausführungsformen 1 bis 25, wobei die erste Aussparung und/oder die zweite Aussparung die Form eines Halbzylinders, eines Zylinders, eines Halbovalzylinders, eines Ovalzylinders, eines Quaders, eines Würfels oder eine Kombination davon aufweist.
27. Kapillarhalter nach Ausführungsform 26, wobei die erste Aussparung und/oder die zweite Aussparung die Form eines Halbovalzylinder und/oder eines Ovalzylinders aufweist, und wobei ein gerader Abschnitt des Halbovalzylinders und/oder des Ovalzylinders parallel zur Längsachse des Hohlkörpers ist.
28. Kapillarhalter nach einer der Ausführungsformen 1 bis 27, wobei die erste Aussparung und/oder die zweite Aussparung derart ausgebildet ist, dass der Mantel des Hohlkörpers und/oder der Mantel des Fixierkörpers einen Vorsprung aufweist.
29. Vorrichtung zur Abnahme einer Flüssigkeitsprobe, insbesondere einer Blutprobe, aufweisend den Kapillarhalter nach einer der Ausführungsformen 1 bis 28 und eine Kapillare.
30. Verfahren zur Fixierung einer Kapillare mit dem Kapillarhalter nach einer der Ausführungsformen 1 bis 28, umfassend die Schritte
   (a) Bereitstellen des Kapillarhalters in einem Aufnahmezustand,
   (b) Aufnehmen eines Abschnitts der Kapillare in der ersten Aussparung, und
   (c) Bewegen des Fixierkörpers relativ zum Hohlkörper, um den Kapillarhalter in einen Fixierzustand zu bringen.
31. Verfahren zum Abnehmen einer Flüssigkeitsprobe, insbesondere Blut, umfassend die Schritte
   (a) bis (c) gemäß Ausführungsform 30, und
   (d) Abnehmen der Flüssigkeitsprobe mit der Kapillare.
32. Verfahren nach Ausführungsform 31, weiters umfassend Schritt (e): Einfüllen der abgenommenen Flüssigkeitsprobe in ein Aufnahmegefäß.

Die Erfindung wird nachstehend anhand von Figurenbeschreibungen einiger Ausführungsformen weiter erläutert. Diese Ausführungsformen sind besonders bevorzugte Ausführungsbeispiele, auf die die Erfindung jedoch nicht beschränkt sein soll.
Fig. 1a zeigt einen Kapillarhalter in einem Aufnahmezustand.
Fig. 1b zeigt den Kapillarhalter 1 der Fig. 1a im Aufnahmezustand, wobei verdeckte Linien strichliert dargestellt sind.
Fig. 1c stellt einen Ausschnitt der Fig. 1b vergrößert dar.
Fig. 2a zeigt eine Vorrichtung 23 zur Abnahme einer Flüssigkeitsprobe in einem Fixierzustand.
Fig. 2b zeigt die Vorrichtung der Fig. 2a im Fixierzustand, wobei verdeckte Linien strichliert dargestellt sind.

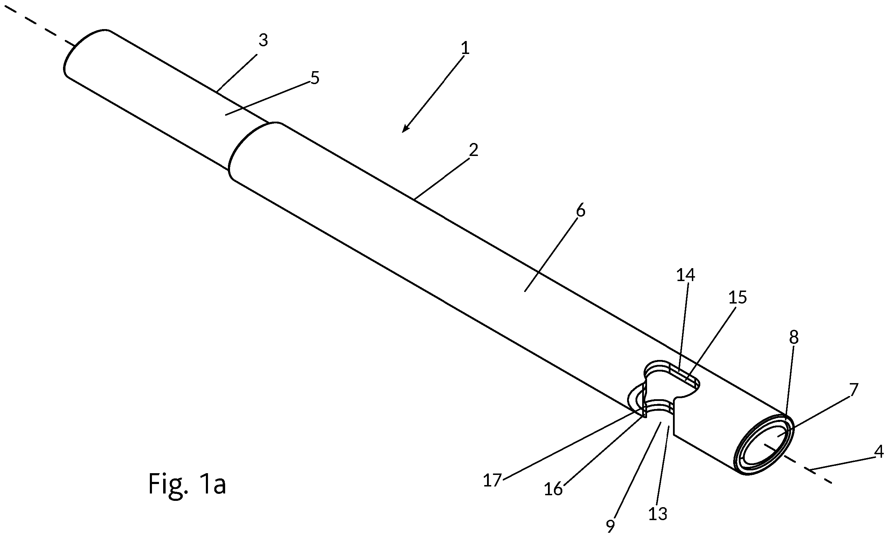

In Fig. 1a ist ein Kapillarhalter 1 in einem Aufnahmezustand dargestellt, dieser weist einen länglichen, zylinderförmigen Hohlkörper 2 auf. Weiters weist der Kapillarhalter 1 einen länglichen, zylinderförmigen Fixierkörper 3 auf, der länger als der Hohlkörper 2 ist und teilweise im Hohlkörper 2 angeordnet ist. Der Hohlkörper 2 und der Fixierkörper 3 weisen eine konstante Querschnittsfläche über ihre Länge auf. Der Fixierkörper 3 ist entlang einer Längsachse 4 des Hohlkörpers 2 bewegbar, wobei diese Längsachse 4 mit einer Längsachse des Fixierkörpers 3 zusammenfällt. Eine Außenseite eines Mantels 5 des Fixierkörpers 3 ist eingerichtet, zumindest teilweise an einer Innenseite eines Mantels 6 des Hohlkörpers 2 anzuliegen. Der Fixierkörper 3 ist hohl und weist geschlossene Endflächen 7 auf, wobei eine der Endflächen 7 im Aufnahmezustand in derselben Ebene liegt wie eine Endfläche 8 des Hohlkörpers 2.

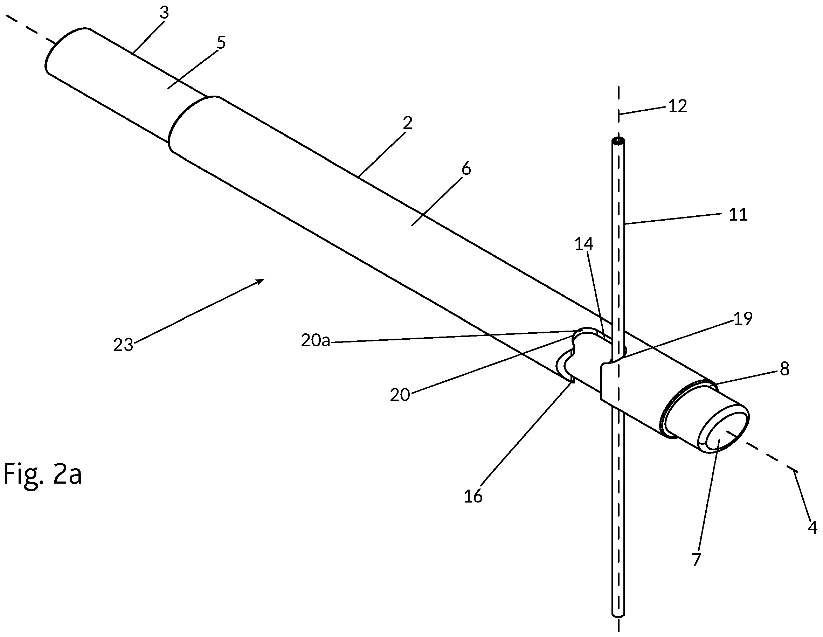

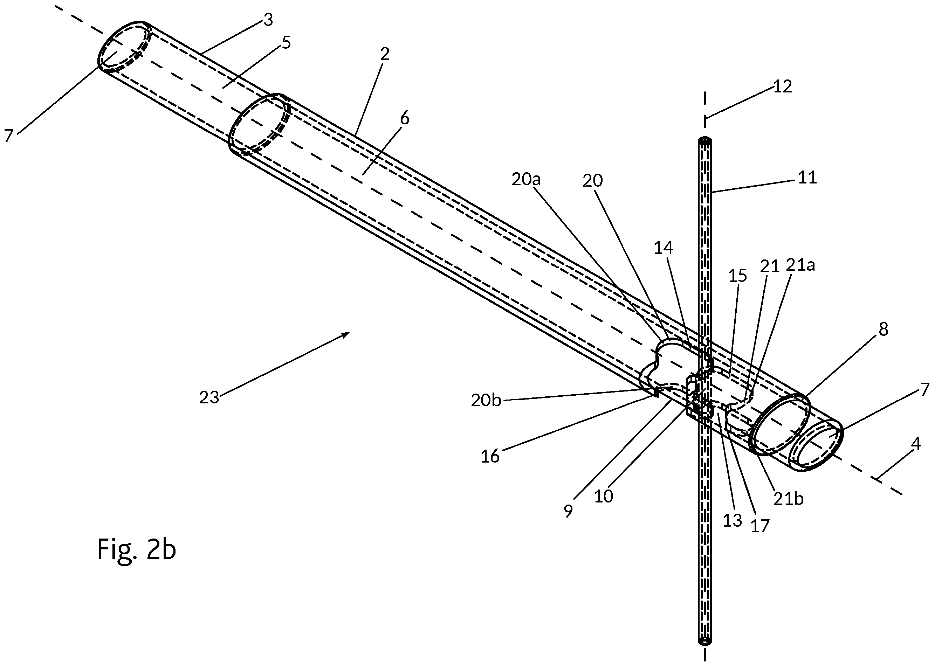

Wie aus Fig. 1a auch ersichtlich ist, weist der Mantel 6 des Hohlkörpers 2 eine von Enden des Hohlkörpers 2 beabstandete, erste Aussparung 9 auf, die eingerichtet ist, einen Abschnitt 10 einer Kapillare 11 (dargestellt in Fig. 2b) derart aufzunehmen, dass eine Längsachse 12 (dargestellt in Fig. 2a-b) der Kapillare im Wesentlichen senkrecht zur Längsachse 4 des Hohlkörpers 2 ist. Der Fixierkörper 3 weist eine zweite Aussparung 13 auf, die eingerichtet ist, einen Teil des in der ersten Aussparung 8 aufgenommenen Abschnitts 10 der Kapillare 11 aufzunehmen. Die erste Aussparung 9 und die zweite Aussparung 13 korrespondieren miteinander und sind im Aufnahmezustand bündig übereinanderliegend angeordnet. Die beiden Aussparungen 9, 13 erstrecken sich in radialer Richtung über zumindest die Hälfte der Querschnittsfläche des Fixierkörpers 3. Weiters weisen die beiden Aussparungen 9, 13 jeweils die Form eines Ovalzylinders auf, wobei ein gerader Abschnitt 14, 15 jedes Ovalzylinders parallel zur Längsachse 4 des Hohlkörpers ist. Die beiden Aussparungen 9, 13 sind derart ausgebildet, dass der Mantel 6 des Hohlkörpers 2 und der Mantel 5 des Fixierkörpers 3 jeweils einen beidseitigen Vorsprung 16, 17 aufweisen. Durch diese Vorsprünge 16, 17 kann die Kapillare 11 im Fixierzustand (siehe Fig. 2a-b) zusätzlich fixiert werden, da sie besser daran gehindert werden kann, aus dem Kapillarhalter 1 herauszufallen oder herausgedrückt zu werden.

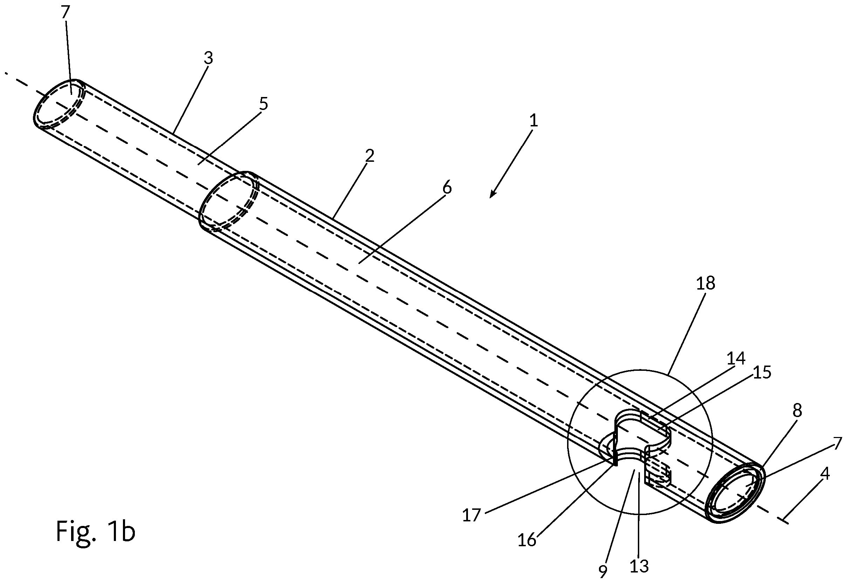

Fig. 1b zeigt den Kapillarhalter 1 der Fig. 1a im Aufnahmezustand, wobei verdeckte Linien strichliert dargestellt sind.

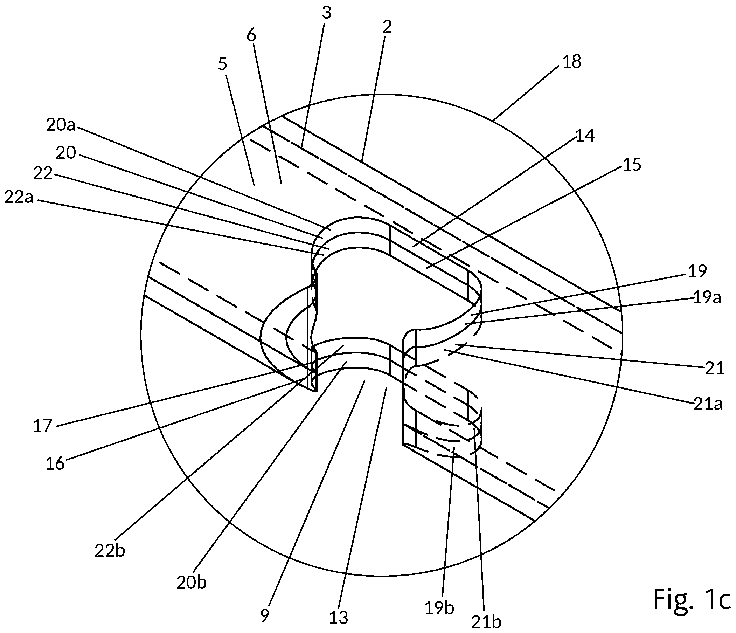

Fig. 1c stellt einen Ausschnitt 18 der Fig. 1b vergrößert dar. Daraus ist ersichtlich, dass der Kapillarhalter 1 zwei Gegenflächen 19, 20 aufweist, die an einander gegenüberliegenden Seiten der durch die erste Aussparung 9 im Mantel 6 des Hohlkörpers 2 gebildeten Fläche angeordnet sind. Die erste Gegenfläche 19 liegt zumindest teilweise in einer ersten Ebene und die zweite Gegenfläche 20 liegt zumindest teilweise in einer zweiten Ebene, wobei die erste Ebene und die zweite Ebene senkrecht zur Längsachse 4 des Hohlkörpers 2 und parallel zueinander sind.

Jede der Gegenflächen 19, 20 weist zwei Teilbereiche 19a-b, 20a-b auf, die an einander gegenüberliegenden Seiten an einer durch die erste Aussparung 9 im Mantel 6 des Hohlkörpers 2 gebildeten Fläche angeordnet sind, wobei die Teilbereiche 19a-b sowie die Teilbereiche 20a-b jeweils zumindest teilweise in einer gemeinsamen Ebene liegen, die senkrecht zur Längsachse 4 des Hohlkörpers 2 ist.

Wie Fig. 1a auch zeigt, sind an einander gegenüberliegenden Seiten der durch die zweite Aussparung 13 im Fixierkörper 3 gebildeten Fläche zwei Fixierflächen 21, 22 angeordnet, wobei die erste Fixierfläche 21 zumindest teilweise in einer ersten Ebene liegt, wobei die zweite Fixierfläche 22 zumindest teilweise in einer zweiten Ebene liegt, und wobei die erste Ebene und die zweite Ebene senkrecht zur Längsachse 4 des Hohlkörpers 2 und parallel zueinander sind.

Jede der zwei Fixierflächen 21, 22 ist eingerichtet, am in der ersten Aussparung 9 aufgenommenen Abschnitt 10 der Kapillare 11 derart anzuliegen, dass die Kapillare 11 durch eine über die jeweilige Fixierfläche 21, 22 auf sie wirkende Kraft an eine von zwei durch die erste Aussparung 9 im Mantel 6 des Hohlkörpers 2 gebildete Gegenflächen 19, 20 gedrückt wird, um die Kapillare 11 zwischen einer der Fixierflächen 21, 22 und einer der Gegenflächen 19, 20 zu fixieren.

Jede Fixierfläche 21, 22 weist zwei Teilbereiche 21a-b, 22a-b auf, die an einander gegenüberliegenden Seiten der durch die zweite Aussparung 13 im Fixierkörper 3 gebildeten Fläche angeordnet sind, wobei die Teilbereiche 21a-b sowie die Teilbereiche 22a-b jeweils zumindest teilweise in einer gemeinsamen Ebene liegen, die senkrecht zur Längsachse 4 des Hohlkörpers 2 ist.

Fig. 2a zeigt eine Vorrichtung 23 zur Abnahme einer Flüssigkeitsprobe in einem Fixierzustand, aufweisend den Kapillarhalter 1 der Fig. 1a-c und eine Kapillare 11. Die Kapillare 11 ist derart in der ersten Aussparung 9 aufgenommen, dass eine Längsachse 12 der Kapillare 11 im Wesentlichen senkrecht zu einer Längsachse 4 des Hohlkörpers 2 ist. Weiters liegt im Fixierzustand die Fixierfläche 21 am in der ersten Aussparung 9 aufgenommenen Abschnitt 10 der Kapillare 11 derart an, dass die Kapillare 11 durch eine über die Fixierfläche 21 auf sie wirkende Kraft, insbesondere Normalkraft, an die durch die erste Aussparung 9 im Mantel 6 des Hohlkörpers 2 gebildete Gegenfläche 19 gedrückt wird.

Fig. 2b zeigt die Vorrichtung 23 der Fig. 2a im Fixierzustand, wobei verdeckte Linien strichliert dargestellt sind. Zur besseren Übersichtlichkeit sind hier nicht alle Bezugszeichen dargestellt.

## Patentansprüche

1. Kapillarhalter (1), aufweisend
einen länglichen Hohlkörper (2), und
einen länglichen Fixierkörper (3), der zumindest teilweise im Hohlkörper (2) angeordnet ist,
wobei der Fixierkörper (3) entlang der Längsachse (4) des Hohlkörpers (2) bewegbar ist,
wobei ein Mantel (6) des Hohlkörpers (2) eine erste Aussparung (9) aufweist, die eingerichtet ist, einen Abschnitt (10) einer Kapillare (11) derart aufzunehmen, dass eine Längsachse (12) der Kapillare (11) im Wesentlichen senkrecht zu einer Längsachse (4) des Hohlkörpers (2) ist, und
wobei der Fixierkörper (3) eine Fixierfläche (21, 22) aufweist, die eingerichtet ist, am in der ersten Aussparung (9) aufgenommenen Abschnitt (10) der Kapillare (11) derart anzuliegen, dass die Kapillare (11) durch eine über die Fixierfläche (21, 22) auf sie wirkende Kraft an eine durch die erste Aussparung (9) im Mantel (6) des Hohlkörpers (2) gebildete Gegenfläche (19, 20) gedrückt wird, um die Kapillare (11) zwischen der Fixierfläche (21, 22) und der Gegenfläche (19, 20) zu fixieren.

2. Kapillarhalter (1) nach Anspruch 1, wobei eine Außenseite eines Mantels (5) des Fixierkörpers (3) eingerichtet ist, zumindest teilweise an einer Innenseite des Mantels (6) des Hohlkörpers (2) anzuliegen.

3. Kapillarhalter (1) nach Anspruch 1 oder 2, wobei der Hohlkörper (2) und/oder der Fixierkörper (3) einen Karton enthalten.

4. Kapillarhalter (1) nach einem der Ansprüche 1 bis 3, wobei der Hohlkörper (2) und/oder der Fixierkörper (3) eine ovale Querschnittsfläche aufweisen.

5. Kapillarhalter (1) nach einem der Ansprüche 1 bis 4, aufweisend zwei Gegenflächen (19, 20), die an einander gegenüberliegenden Seiten der durch die erste Aussparung (9) im Mantel (6) des Hohlkörpers (2) gebildeten Fläche angeordnet sind, wobei die erste Gegenfläche (19) zumindest teilweise in einer ersten Ebene liegt, wobei die zweite Gegenfläche (20) zumindest teilweise in einer zweiten Ebene liegt, und wobei die erste Ebene und die zweite Ebene senkrecht zur Längsachse (4) des Hohlkörpers (2) und parallel zueinander sind.

6. Kapillarhalter (1) nach einer der Ansprüche 1 bis 5, wobei der Fixierkörper (3) eine zweite Aussparung (13) aufweist, die eingerichtet ist, einen Teil des in der ersten Aussparung (9) aufgenommenen Abschnitts (10) der Kapillare (11) aufzunehmen.

7. Kapillarhalter (1) nach Anspruch 6, aufweisend zwei Fixierflächen (21, 22), die an einander gegenüberliegenden Seiten der durch die zweite Aussparung (13) im Fixierkörper (3) gebildeten Fläche angeordnet sind, wobei die erste Fixierfläche (21) zumindest teilweise in einer ersten Ebene liegt, wobei die zweite Fixierfläche (22) zumindest teilweise in einer zweiten Ebene liegt, und wobei die erste Ebene und die zweite Ebene senkrecht zur Längsachse (4) des Hohlkörpers (2) und parallel zueinander sind.

8. Kapillarhalter (1) nach einem der Ansprüche 1 bis 7, wobei sich die erste Aussparung (9) und/oder die zweite Aussparung (13) in radialer Richtung über zumindest die Hälfte der Querschnittsfläche des Fixierkörpers (3) erstrecken.

9. Vorrichtung (23) zur Abnahme einer Flüssigkeitsprobe, aufweisend den Kapillarhalter (1) nach einem der Ansprüche 1 bis 8 und eine Kapillare (11).

10. Verfahren zur Fixierung einer Kapillare (11) mit dem Kapillarhalter (1) nach einem der Ansprüche 1 bis 8, umfassend die Schritte
(a) Bereitstellen des Kapillarhalters (1) in einem Aufnahmezustand,
(b) Aufnehmen eines Abschnitts (10) der Kapillare (11) in der ersten Aussparung (9), und
(c) Bewegen des Fixierkörpers (3) relativ zum Hohlkörper (2), um den Kapillarhalter (1) in einen Fixierzustand zu bringen.
